# EUROPÄISCHE PATENTSCHRIFT

(11) **EP 0 392 038 B1**
(45) Veröffentlichungstag und Bekanntmachung des Hinweises auf die Patenterteilung: **20.07.1994**
(21) Anmeldenummer: 89106363.8
(22) Anmeldetag: 11.04.1989
(51) Int. Cl.: C07D 417/12, A61K 31/425, A61K 31/50

(54) **Acylureidoaminothiazolyl-Abkömmlinge und Verfahren zu deren Herstellung**
Acylureidoaminothiazolyl derivatives and process for their preparation
Dérivés d'acylureido thiazoles et procédé de préaparation

(43) Veröffentlichungstag der Anmeldung: 17.10.1990
(73) Patentinhaber: TECHNOLOGITCHEN KOMBINAT ZA PROMISHLENA MIKROBIOLOGIA, Razgrad (BG)
(72) Erfinder: Atanassova, Tashka Koleva, Block 435-A Sofia (BU); Nakov, Anton Ivanov, Sofia (BU); Petkova, Lyudmila Vutova, Dr., Sofia (BU); Ivanova, Zoya Tantcheva, Sofia (BU); Mondeshka, Donka Minkova, Sofia (BU)
(74) Vertreter: von Füner, Alexander, Dr.

(56) Entgegenhaltungen:
- EP-A- 0 075 600
- DE-A- 2 120 367
- CHEMICAL ABSTRACTS, Band 103, Nr. 13, 30. September 1985, Seite 623, Zusammenfassung Nr. 104958e, Columbus, Ohio, US;

## Beschreibung

Die Erfindung betrifft Acylureidoaminothiazolyl-Abkömmlinge mit pharmakologischer Wirkung und Verfahren zu deren Herstellung.

Es sind 2-Aminothiazolyl-Abkömmlinge bekannt, die als N-acylen Substituent einen Rest mit 1 bis 4 Kohlenstoffatomen und eine immunmodulatore Wirkung haben, so daß sie gegen immune Erkrankungen recht aktiv wirken (GB-A- 2 110 674, US-A- 4 473 577 und JP-A- 60 13 773).

Der Erfindung liegt die Aufgabe zugrunde, neue 2-Aminothiazolyl-Abkömmlinge mit pharmakologischer Wirkung bereitzustellen und ein Verfahren zu deren Herstellung zu entwickeln.

Erfindungsgemäß werden neue Acylureidoaminothiazolyl-Abkömmlinge der allgemeinen Formel: 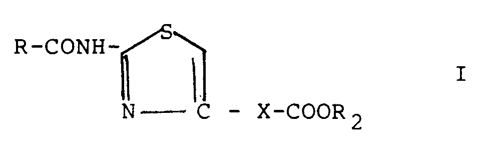
vorgeschlagen, worin R eine Gruppe der Formel: 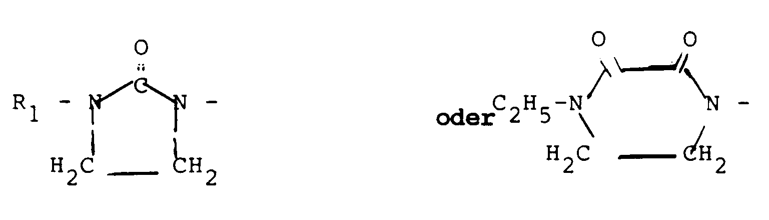
bedeutet, R₁ für Wasserstoff oder die Gruppe CH₃SO₂ steht; R₂ Wasserstoff oder einen niederen Alkylrest bedeutet und X für die Gruppe 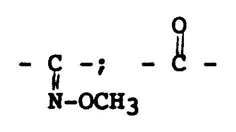
oder -CH₂ steht.

Die neuen Acylureidoaminothiazolyl-Abkömmlinge werden durch Acylierung von Verbindungen der allgemeinen Formel: 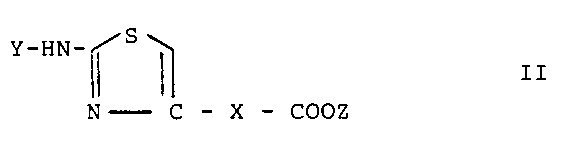
erhalten, worin X die obigen Bedeutungen hat, während Z für einen niederen Alkylrest oder -Si(CH₃)₃ und Y für -H oder -Si(CH₃)₃ steht, mit Verbindungen der allgemeinen Formel

RCOCl III

worin R die obige Bedeutung hat, in einem nichtwässerigen Medium in Anwesenheit eines Chlorwasserstoffakzeptors bei einer Temperatur des Reaktionsmediums von 0°C bis zur Siedetemperatur des entsprechenden Lösungsmittels, in welchem die Acylierung erfolgt.

Als organisches Lösungsmittel für die Acylierung kann ein halogenierter Kohlenwasserstoff wie Methylenchlorid, Chloroform, Tetrachlormethan, Ester von niedrigen Fettsäuren wie Ethylacetat, Butylacetat, niedrige Dialkylketone, wie Aceton, Methylethylketon, cyclische Ester wie Tetrahydrofuran, Benzol oder Toluol verwendet werden.

In den Fällen, wo für die Acylierung Verbindungen der Formel II eingesetzt werden, wo Y = Z bedeutet, -Si(CH₃)₃ oder Y für Wasserstoff und Z für -Si(CH₃)₃ steht, werden die Verbindungen der Formel II nach bekannten Verfahren für die Silylierung mit beliebigen der Silylierungsagenten, welche in der angewandten Chemie viel verwendet werden, hergestellt.

Als Chlorwasserstoffakzeptoren werden z.B. Triethylamin, Dimethylanilin oder Pyridin oder eine Mischung aus N-Silyl-Abkömmlingen der entsprechenden Amide oder Carbamide und Triethylammoniumchlorid eingesetzt.

Die Reaktionskomponenten können bei der Acylierung in äquimolaren Mengen teilnehmen oder, um die Ausbeute an Endprodukt zu erhöhen und einen sparsamen Verbrauch der Verbindungen der Formel II zu gewähren, kann man die Carbamoylchloride im Überschuß von 0,1 bis 0,3 Mol dosieren, je nach dem organischen Lösungsmittel und den Bedingungen, unter welchen der Prozeß verläuft. Die nicht umgesetzten Carbamoylchloride werden während der Isolierung der entsprechenden Acylureidoaminothiazolyl-Abkömmlinge in neutrale, leicht lösbare Produkte umgewandelt, was die Isolierung und Reinigung des Endprodukts erleichtert.

Die Acylierung der Verbindungen der Formel II kann in einem breiten Temperaturbereich von 0°C bis zur Siedetemperatur des entsprechenden Lösungsmittels während 2 bis 28 Stunden erfolgen. Die niedrigen Temperaturen verursachen eine Verringerung der Reaktionsgeschwindigkeit.

Die Bedingungen, unter denen die Acylierung erfolgt, sowie die Isolierung der erhaltenen Aminothiazolyl-Abkömmlinge gewährleisten die Aufrechterhaltung der Syn-Konfiguration der Ausgangsverbindung der Formel II, worin "X" für 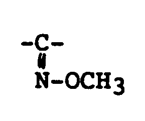
steht.

Die neuen Acylureidoaminothiazolyl-Abkömmlinge besitzen eine hohe Wirkung gegen Entzündungen.

### Beispiel 1

### Methylester von 2-[2-(Imidazolidin-1-carboxamido)-thiazol-4-yl]-2-syn-methoxyimino-Essigsäure.

Zu 60 ml Methylenchlorid werden 4,3 g (20 mMol) Methylester von 2-(2-Aminothiazol-4-yl)-2-methoxyimino-Essigsäure zugegeben. Man kühlt auf 0 bis 5°C ab und setzt 3,3 ml Triethylamin und 3,5 g (24 mMol) 1-Chlorcarbonylimidazolidon-2 zu. Die Reaktionsmischung wird 4 Stunden lang am Rückflußkühler erhitzt. Dan kühlt man bis auf Zimmertemperatur ab und filtriert. Das Filtrat wird zuerst mit einer HCl-Lösung, dann mit einer Natriumbicarbonatlösung und zuletzt mit Wasser gewaschen. Es wird mit wasserfreiem Natriumsulfat getrocknet und unter Vakuum konzentriert. Den Niederschlag erhält man durch Zufügen von Diisopropylether. Man erhält 5,7 g des Endprodukts.

| | | | |
|---|---|---|---|
| C₁₁H₁₃N₅O₅ berechnet, %: | C 40,36; | H 4,00; | N 21,40. |
| (327,31) gefunden, %: | C 40,38; | H 4,11; | N 21,44. |

¹NKMR-Spektrum (COCl₃):
- δ(ppm):: 3,18, 3,60 (4H,m)-4 CH₂ und 5 CH₂; 3,65 (3H,s) und 3,85(3H, s)-COOCH₃ und -N-OCH₃
7.04 (1H,s)-H₅; 7,70 (1H breit) -2 CONH; 11,10 (1H,s breit) -2 CONH;
- IR-Spektrum (KBr): 3300, 3180, 1720, 1690, 1525, 1260, 1020 cm⁻¹.

### Beispiel 2

### Methylester von 2-[-(4-Ethyl-2,3-dioxo-1-piperazin-carboxamido)-thiazol-4-yl]-2-syn-methoxyimino-essigsäure.

Diese Verbindung wird erfindungsgemäß nach der Verfahrensweise von Beispiel 1 erhalten, indem man 4,3 g (20 mMol) Methylester von 2-(2-Aminothiazol-4-yl)-2-methoxyiminoessigsäure, 3,6 ml (26 mMol) Triethylamin und 5,3 g (26 mMol) 4-Ethyl-2,3-dioxo-piperazincarbonylchlorid miteinander reagieren läßt. Das Reaktionsgemisch wird nach Konzentrierung unter Vakuum auf eine chromatographishe Kolonne mit Silikagel geleitet. Man eluiert mit einem Gemisch, bestehend aus Ethylacetat und Tetrahydrofuran. Das Eluat wird unter Vakuum konzentriert. Die Ausbeute an Endprodukt beträgt 5,8 g.

| | | | |
|---|---|---|---|
| C₁₄H₁₇O₆S, berechnet, %: | C 43,86 | H 4,47 | N 18,27 |
| (383,38) gefunden, %: | C 43,77 | H 4,39 | N 18,31 |

¹NKMR-Spektrum (COCl₃):
- δ(ppm):: 1,14 (3H,t) and 3,36 (2Hq) -N-C₂H₅; 3,45 (2H,m) und 4,00 (2H,m)-5 CH₂ und 6-CH₂; 3,73 (3H,s) und 3,85 (3H,s) -N-OCH₃ und COOCH₃; 6,94 (1H,s) -5′-H; 11,82 (1H,s breit) - wird mit D₂ -2-CONH
umgetauscht.
- IR-Spektrum (KBr): 3400, 3180, 1730, 1690, 1438, 1525, 1720, 1280, 1038 cm⁻¹.

### Beispiel 3

### 2-[2-(Imidazolidin-1-carboxamido)-thiazol-4-yl]-2-syn-methoxyimino-Essigsäure.

Zur Suspension von 4,0 g (20 mMol) 2-(2-Aminothiazol-4-yl)-2-methoxyimino-Essigsäure in 30 ml Chloroform werden 67 ml (44 mMol) Triethylamin und 5,6 ml (44 mMol) Trimethylchlorsilan zugefügt. Das Reaktionsgemisch wird unter Rühren 3 h lang am Rückflußkühler erhitzt und danach filtriert. Zum bis auf -5°C abgekühlten Filtrat werden 2,9 ml (21 mMol) Triethylamin und 3,1 g (21 mMol) 1-Chlorcarbonylimidazolidon-2 zugesetzt. Das Reaktionsgemisch wir 8 h lang bei Zimmertemperatur gerührt. Man filtriert, setzt 30 ml Wasser zu und rührt noch 20 min lang. Der pH-Wert des Gemisches wird auf 1,5 eingestellt und die Schichten werden getrennt. Das Chloroformkonzentrat wird zuerst mit einer HCl-Lösung und dann mit Wasser gewaschen. Man trocknet mit wasserfreiem Magnesiumsulfat und konzentriert unter Vakuum. Man erhält 5 g des Endprodukts.

| | | | |
|---|---|---|---|
| C₁₀H₁₁N₅O₅ berechnet, %: | C 38,34; | H 3,54; | N 22,36 |
| (313,29) gefunden, %: | C 38,39; | H 3,61; | N 22,41. |

¹NKMR-Spektrum (DMSO-d₆)
- δ(ppm):: 3,26 und 3,72 (4H,m) - 4′-CH₂ und 5′-CH₂; 3,72 (3H,s), -N-OCH₃; 7,16 (1H,s) - H-5; 7,82 (1H,s), wird mit D₂O -2′-CONH umgetauscht;
11,24 (1H,s breit) - wird mit D₂O -2-CONH umgetauscht.

### Beispiel 4

### 2-[2-(4-Ethyl-2,3-dioxo-1-piperazin-carboxamido)-thiazol-4-yl]-2-syn-methoxyimino-Essigsäure.

Zur Suspension von 4,0 g (20 mMol) 2-(2-Aminothiazol-4-yl)-2-methoxyimino-Essigsäure in 15 ml Methylenchlorid werden 4,6 ml (22 mMol) Hexamethyldisilazan zugesetzt. Das Gemisch wird am Rückflußkühler unter Rühren während 4 h erhitzt und danach bis auf -50°C abgekühlt. Zur abgekühlten Mischung werden 3,0 ml (22 mMol) Triethylamin und 4,5 g (22 mMol) 4-Ethyl-2,3-dioxo-1-piperazincarbonylchlorid zugesetzt. Man rührt bei Zimmertemperatur während 6 h, filtriert und setzt 30 ml Wasser zu. Es wird 20 min lang hydrolysiert und dann wird der pH auf 1,5 eingestellt. Die Schichten werden getrennt. Die organische Schicht wird unter Vakuum konzentriert und das Rohprodukt einer chromatographischen Kolonne mit Silikagel zugeführt. Nach Eluieren mit Chloroform:Tetrahydrofuran (18:2) und Konzentrierung des Eluats werden 5,6 g des Produkts erhalten:

| | | | |
|---|---|---|---|
| C₁₃H₁₅N₅O₆S berechnet, %: | C 42,28; | H 4,09 | N 18,96 |
| (369,38) gefunden, %: | C 42,25; | H 4,12 | N 18,77 |

¹NKMR-Spektrum (DMSO-d₆):
- δ(ppm):: 1,17 (3H,t) und 3,40 (2H,q)-N-C₂H₅; 3,50 (2H,m) 4,08 (2H,m)-5′-CH₂ und 6′-CH₂; 3,72 (3H,s)-N-OCH₃ 7,02(1H,s)-H-5; 11,6 (1H,s breit)-wird mit D₂O -2-CONH
umgestauscht.

### Beispiel 5

### Ethylester von 2-[2-(Imidazoliden-1-carboxamido)-thiazol-4-yl]-Essigsäure.

Diese Verbindung wird nach dem Verfahren von Beispiel 1 durch Umsetzen von 1,9 g (10 mMol) Ethyl-2-(2-aminothiazol-4-yl)-acetat mit 1,6 g (11 mMol) 1-Chlorcarbonylimidazolidon-2- in Anwesenheit von 1,5 ml (11 mMol) Triethylamin als Chlorwasserstoffakzeptor. Man erhält 2,2 g des Endprodukts.

| | | | |
|---|---|---|---|
| C₁₁H₁₄N₄O₄S berechnet, %: | C 44,29; | H 4,73; | N 18,78. |
| (208,31) gefunden, % | C 44,32; | H 4,80; | N 18,82. |

¹NKMR-Spektrum (DMSO-d₆):
- δ(ppm):: 1,10 (3H,t) und 3,9 (2H,q)-OC₂H₅ 3,47 (2H,s) -4-CH₂; 3,24 und 3,69 (4H,m)-4-CH₂ und 5′CH₂ 6,67 (1H,s)-H; 7,75 (1H,s breit) und 11,13 (1H,s breit) - wird mit D₂O -2′CONH und 2-CONH umgetauscht.
- IR-Spektrum (KBr): 3295, 3175, 1735, 1720, 1680, 1410, 1425, 1435, 1350, 1250, 1020 cm⁻¹.

### Beispiel 6

### Ethylester von 2-[2-(4-Ethyl-2,3-dioxo-1-piperazin-carboxamido)-thiazol-4-yl]-Essigsäure.

Diese Verbindung wird nach dem Verfahren von Beispiel 2 durch Umsetzen von 1,9 g (10 mMol) Ethyl-2-(2-aminothiazol-4-yl)acetat mit 2,7 g (13 mMol) 4-Ethyl-2,3-dioxo-1-piperazincarbonylchlorid in Anwesenheit von 1,8 ml (13 mMol) Triethylamin als Chlorwasserstoffakzeptor erhalten. Man erhält 2,9 g Endprodukt.

| | | | |
|---|---|---|---|
| C₁₄H₁₈N₄O₅S berechnet, %: | C 47,45; | H 5,12; | N 15,81 |
| (354,34) gefunden, %: | C 47,48; | H 5,11; | N 15,79. |

¹NKMR-Spektrum (DMSO-d₆)
- δ(ppm):: 1,03(3H,t) und 3,27(2H,q)-N-C₂H₅; 1,12(3H,t) und 3,92(2H,q)-COOC₂H₅; 3,50 und 3,88(4H,m)-5′-CH₂ und 6′-CH₂; 3,54 (2H,s)-4-CH₂; 6,81 (2H,s); 11,28(1H,s breit)-wird mit D₂O -CONH umgetauscht.
- IR-Spektrum (KBr): 3390, 1710, 1680, 1520, 1410, 1425, 1435, 1350, 1285, 1138 cm⁻¹

### Beispiel 7

### 2-[2-(Imidazolidin-1-carboxamido)-thiazol-4-yl]-Essigsäure.

Diese Verbindung wird wie in Beispiel 3 beschrieben durch Silylieren von 3,2 g (20 mMol) 2-(2-Aminothiazol-4-yl)-Essigsäure mit 5,6 ml (44 mMol) Triethylchlorsilan in Anwesenheit von 1,6 ml (44 mMol) Triethylamin als Chlorwasserstoffakzeptor und nachfolgendes Acylieren des erhaltenen Silylesters mit 3,1 g (21 mMol) 1-Chlorcarbonyl-imidazolidon-2 erhalten. Man erhält 3,9 g des Endprodukts.

| | | | |
|---|---|---|---|
| C₉H₁₀N₄O₄S berechnet, %: | C 39,00; | H 3,73; | N 20,73 |
| (270,26) gefunden, %: | C 40,01; | H 3,78; | N 20,76 |

¹NKMR-Spektrum (DMSO-d₆)
- δ(ppm):: 3,53 (3H,s), 3,28(2H,q) und 3,72 (4H,m)-4′-CH₂ und 5′-CH₂, 6,67(1H,s)-H-5; 7,78(1H,Br) und 11,13 (1H,s breit) - wird mit D₂O -2′-CONH und 2-CONH umgetauscht.

### Beispiel 8

### 2-[2-(4-Ethyl-2,3-dioxo-1-piperazincarboxamido)-thiazol-4-yl]-Essigsäure.

Diese Verbindung wird nach dem Verfahren von Beispiel 4 durch Silylieren von 3,2 g (20 mMol) 2-(-2-Aminothiazol-4-yl)-Essigsäure mit 4,6 ml (22 mMol) Hexamethyldisilazan und nachfolgendes Acylieren des so erhaltenen Silylesters mit 4,5 g (22 mMol) 4-Ethyl-2,3-dioxo-1-piperazino-carbonylchlorid in Anwesenheit eines Chlorwasserstoffakzeptors erhalten. Man erhält 4,8 g des Endprodukts.

| | | | |
|---|---|---|---|
| C₁₂H₁₄N₄O₅S berechnet, %: | C 44,17; | H 4,32; | N 17,17 |
| (326,32) gefunden, %: | C 44,21; | H 4,35; | N 17,27 |

¹NKMR-Spektrum (DMSO-d₆)
- δ(ppm):: 1,07(3H,t); 3,25(2H,q)-N-C₂H₅; 3,35 und 3,78 (4H,m)-5 CH₂ und 6′CH₂; 3,54(2H,s)-4-CH₂; 6,81(2H,s)-H-5; 11,27(1H,s breit)- wird mit D₂O -CONH umgestauscht.

### Beispiel 9

### Ethylester von 2-[2-(Imidazolidin-1-carboxamido)-thiazol-4-yl]-Glyoxalsäure.

Zu 50 ml Tetrahydrofuran werden 4,0 g (20 mMol) Ethylester von 2-(2-Aminothiazol-4-yl)-glyoxalsäure zugesetzt. Man kühlt bis auf 0 bis 5°C ab und fügt 3,2 ml Triethylamin und 3,3 g (23 mMol) 1-Chlorcarbonylimidazolidon-2 zu. Das Reaktionsgemisch wird 5 bis 6 h lang am Rückflußkühler erhitzt. Man kühlt bis auf Zimmertemperatur ab und filtriert. Der Rest wird in 50 ml Chloroform gelöst. Die Chloroformlösung wird zuerst mit einer HCl-Lösung, dann mit einer Na₂CO₃-Lösung und zuletzt mit Wasser gewaschen. Man trocknet mit wasserfreiem Na₂SO₄ und konzentriert unter Vakuum. Man fällt durch Zusatz von Diisopropylester. Die Ausbeute an Endprodukt beträgt 4,6 g.

| | | | |
|---|---|---|---|
| C₁₁H₁₂N₄O₅S berechnet, %: | C 42,30; | H 3,87; | N 17,94 |
| (312,30) gefunden, %: | C 42,43; | H 3,92; | N 18,03 |

¹NKMR-Spektrum (DMSO-d₆)
- δ(ppm):: 1,22(3H,t) und 4,19(2H,q)-OC₂H₅; 3,24 und 3,27(4H,m)-4′-CH₂ und 5′-CH₂; 8,15(1H,s)-H-5; 7,90(1Hm,s breit) und 11,58(1H,s breit) - wird mit D₂O - 2′-CONH und 2-CONH umgetauscht.

### Beispiel 10

### Ethylester von 2-/2-(4-Ethyl-2,3-dioxo-1-piperazincarboxamido)-thiazol-4-yl/-Glyoxalsäure.

Diese Verbindung wird nach dem Verfahren von Beispiel 9 durch Umsetzen von 4,0 g (20 mMol) Ethylester von 2-(2-Aminothiazol-4-yl)-Glyoxalsäure, 3,3 ml (24 mMol) Triethylamin und 4,9 g (24 mMol) 4-Ethyl-2,3-dioxo-1-piperazin-carbonylchlorid erhalten. Die Chloroformlösung wird nach Konzentrierung unter Vakuum einer chromatographischen Kolonne mit Silikagel zugeleitet. Das Eluat wird unter Vakuum konzentriert. Man erhält 6,1 g des Endprodukts.

| | | | |
|---|---|---|---|
| C₁₄H₁₆N₄O₆S berechnet, %: | C 45,65; | H 4,38; | N 15,21 |
| (368,36) gefunden, %: | C 45,74; | H 4,43; | N 15,17 |

¹NKMR-Spektrum (DMSO-d₆):
- δ(ppm):: 1,05(3H,t) und 3,30(2H,q)-N-C₂H₅ und 1,25 (3H,t) und 4,18(2H,q)-COOC₂H₆; 3,50(2H,m) und 3,89(2H,m)-5′CH₂ und 6′CH₂; 8,18(1H,s)-H-5; 11,68(1H,s breit) - wird mit D₂O -CONH umgetauscht.

### Beispiel 11

Die Ergebnisse der Antientzündungswirkung (Karagenin-Schwellung) der neuen Acylureidoaminothiazolyl-Derivate gemäß dem Verfahren Winter et al. sind in den Tabellen 1, 2, 3, 4, 5 und 6 dargestellt.

**Tabelle 1**

| Methylester von 2-(2-Imidazolidin-1-carboxyamidothiazol-4-yl)-2-syn-methoxyimino-Essigsäure (Beispiel 1) | | | | | | | |
|---|---|---|---|---|---|---|---|
| Dosis | Ausgangswert | 1h | 2h | 3h | 4h | 5h | 24h |
| 1 mg | 1 | 1,29 | 1,52 | 1,58 | 1,67 | 1,59 | 1,41 |
| | | 129% | 152% | 158% | 167% | 159% | 141% |
| 3 mg | 0,96 | 1,40 | 1,37 | 1,57 | 1,66 | 1,57 | 1,38 |
| | | 146% | 143% | 164% | 173% | 164% | 144% |
| 5 mg | 1,06 | 1,31 | 1,46 | 1,64 | 1,72 | 1,73 | 1,23 |
| | | 123% | 138% | 154% | 166% | 165% | 115% |
| 10 mg | 1,04 | 1,21 | 1,27 | 1,47 | 1,61 | 1,58 | 1,17 |
| | | 116% | 122% | 141% | 155% | 152% | 113% |
| Kontrolle | 0,91 | 1,28 | 1,58 | 1;72 | 1,77 | 1,80 | 1,38 |
| | | 141% | 174% | 189% | 195% | 198% | 152% |

**Tabelle 2**

| Methylester von 2-[2-(4-Ethyl-2,3-dioxo-1-piperazino-carboxamido)-thiazol-4-yl]-2-syn-methoxyimino-Essigsäure (Beispiel 2) | | | | | | | |
|---|---|---|---|---|---|---|---|
| Dosis | Ausgangswert | 1h | 2h | 3h | 4h | 5h | 24h |
| 1 mg | 0,96 | 1,27 | 1,55 | 1,60 | 1,66 | 1,63 | 1,45 |
| | | 132% | 161% | 166% | 172% | 169% | 151% |
| 3 mg | 1,0 | 1,39 | 1,40 | 1,57 | 1,67 | 1,62 | 1,35 |
| | | 139% | 140% | 157% | 167% | 162% | 135% |
| 5 mg | 1,04 | 1,29 | 1,46 | 1,62 | 1,72 | 1,74 | 1,25 |
| | | 124% | 140% | 155% | 165% | 167% | 120% |
| 10 mg | 1,06 | 1,22 | 1,25 | 1,46 | 1,63 | 1,60 | 1,15 |
| | | 115% | 117% | 137% | 154% | 151% | 108% |
| Kontrolle | 0,92 | 1,29 | 1,61 | 1,71 | 1,77 | 1,83 | 1,43 |
| | | 140% | 175% | 185% | 192% | 199% | 155% |

**Tabelle 3**

| Ethylester von 2-[2-(Imidazolidin-1-carboxamido)-thiazol-4-yl]-Essigsäure (Beispiel 5) | | | | | | | |
|---|---|---|---|---|---|---|---|
| Dosis | Ausgangswert | 1h | 2h | 3h | 4h | 5h | 24h |
| 1 mg | 0,87 | 1,23 | 1,36 | 1,39 | 1,53 | 1,47 | 1,16 |
| | | 141% | 156% | 160% | 175% | 169% | 133% |
| 3 mg | 0,91 | 1,29 | 1,18 | 1,25 | 1,45 | 1,30 | 1,18 |
| | | 142% | 130% | 137% | 159% | 143% | 130% |
| 5 mg | 0,94 | 1,08 | 1,20 | 1,31 | 1,43 | 1,49 | 1,14 |
| | | 115% | 128% | 139% | 152% | 159% | 121% |
| 10 mg | 0,95 | 1,06 | 1,20 | 1,33 | 1,46 | 1,54 | 1,07 |
| | | 113% | 126% | 140% | 154% | 162% | 113% |
| Kontr. | 0,91 | 1,28 | 1,58 | 1,72 | 1,77 | 1,80 | 1,38 |
| | | 141% | 174% | 189% | 195% | 198% | 152% |

**Tabelle 4**

| Ethylester von 2-[2-(4-Ethyl-2,3-dioxo-1-piperazin-carboxamido)-thiazol-4-yl]-Essigsäure (Beispiel 6) | | | | | | | |
|---|---|---|---|---|---|---|---|
| Dosis | Ausgangswert | 1h | 2h | 3h | 4h | 5h | 24h |
| 1 mg | 0,90 | 1,25 | 1,34 | 1,40 | 1,52 | 1,50 | 1,12 |
| | | 139% | 149% | 156% | 168% | 165% | 124% |
| 3 mg | 0,92 | 1,30 | 1,30 | 1,32 | 1,47 | 1,38 | 1,12 |
| | | 141% | 141% | 142% | 158% | 148% | 120% |
| 5 mg | 0,94 | 1,10 | 1,23 | 1,34 | 1,43 | 1,50 | 1,10 |
| | | 117% | 129% | 142% | 152% | 160% | 117% |
| 10 mg | 0,95 | 1,08 | 1,20 | 1,36 | 1,42 | 1,52 | 1,10 |
| | | 113% | 126% | 143% | 149% | 160% | 115% |
| Kontr. | 0,93 | 1,31 | 1,62 | 1,75 | 1,80 | 1,85 | 1,85 |
| | | 140% | 174% | 188% | 193% | 199% | 146% |

**Tabelle 5**

| Ethylester von 2-[2-(Imidazolidin-1-carboxamido)-thiazol-4-yl]-Glyoxalsäure (Beispiel 9) | | | | | | | |
|---|---|---|---|---|---|---|---|
| Dosis | Ausgangswert | 1h | 2h | 3h | 4h | 5h | 24h |
| 1 mg | 0,96 | 1,32 | 1,54 | 1,72 | 1,62 | 1,56 | 1,31 |
| | | 135% | 160% | 179% | 169% | 163% | 147% |
| 3 mg | 0,97 | 1,32 | 1,57 | 1,67 | 1,57 | 1,67 | 1,27 |
| | | 136% | 162% | 165% | 162% | 172% | 131% |
| 5 mg | 1,11 | 1,38 | 1,63 | 1,73 | 1,75 | 1,69 | 1,34 |
| | | 124% | 147% | 158% | 158% | 152% | 121% |
| 10 mg | 1,05 | 1,36 | 1,68 | 1,85 | 1,68 | 1,73 | 1,25 |
| | | 130% | 160% | 176% | 160% | 164% | 119% |
| Kontrolle | 1,01 | 1,28 | 1,42 | 1,72 | 1,72 | 1,69 | 1,35 |
| | | 127% | 141% | 171% | 171% | 168% | 134% |

**Tabelle 6**

| Ethylester von 2-[2-(4-Ethyl-2,3-dioxo-1-piperazin-carboxamido)-thiazol-4-yl]-Glyoxalsäure (Beispiel 10) | | | | | | | |
|---|---|---|---|---|---|---|---|
| Dosis | Ausgangswert | 1h | 2h | 3h | 4h | 5h | 24H |
| 1 mg | 0,90 | 1,30 | 1,54 | 1,61 | 1,67 | 1,57 | 1,27 |
| | | 144% | 171% | 179% | 185% | 174% | 141% |
| 3 mg | 0,95 | 1,15 | 1,26 | 1,55 | 1,59 | 1,64 | 1,21 |
| | | 121% | 133% | 163% | 167% | 173% | 127% |
| 5 mg | 0,96 | 1,21 | 1,30 | 1,55 | 1,52 | 1,61 | 1,15 |
| | | 126% | 135% | 161% | 158% | 168% | 120% |
| 10 mg | 0,95 | 1,07 | 1,34 | 1,33 | 1,44 | 1,54 | 1,12 |
| | | 112% | 141% | 140% | 152% | 162% | 118% |
| Kontrolle | 1,01 | 1,28 | 1,42 | 1,72 | 1,72 | 1,69 | 1,35 |
| | | 127% | 141% | 171% | 171% | 168% | 134% |

## Patentansprüche

1. Acylureidoaminothiazolyl-Abkömmlinge der allgemeinen Formel 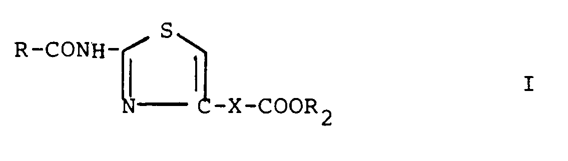 worin R eine Gruppe der Formel 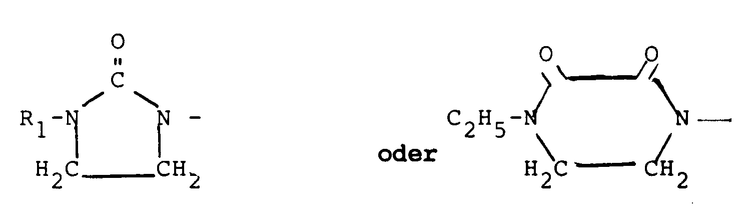 bedeutet und R₁ für Wasserstoff oder CH₃SO₂, R₂ für Wasserstoff oder einen niederen Alkylrest und X für 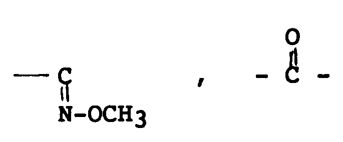 oder -CH₂-
stehen.

2. Verfahren zur Herstellung der Verbindungen nach Anspruch 1, dadurch **gekennzeichnet**, daß man eine Verbindung der allgemeinen Formel 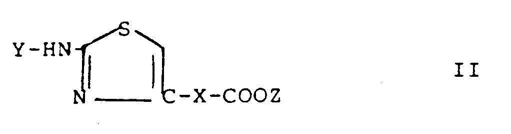 worin X die obigen Bedeutungen hat, Z für einen niederen Alkylrest oder -Si(CH₃)₃ steht und Y für -H oder die Gruppe -Si(CH₃)₃ bedeutet, mit Verbindungen der Formel
RCOCl III
worin R die obigen Bedeutungen besitzt, acyliert.

3. Verfahren nch Anspruch 2, dadurch **gekennzeichnet,** daß man die Acylierung in wasserfreien organischen Lösungsmitteln, wie halogenierten Kohlenwasserstoffen oder Estern von niedrigen Fettsäuren vornimmt.

4. Verfahren nach Anspruch 2, dadurch **gekennzeichnet**, daß man die Acylierung in Anwesenheit eines Chlorwasserstoffakzeptors vornimmt.

5. Verfahren nach Anspruch 2, dadurch **gekennzeichnet,** daß man die Acylierung bei einer Temperatur von 0°C bis zur Siedetemperatur des Lösungsmittels vornimmt.

## Claims

1. Acylureidoaminothiazolyl-derivatives of the general formula 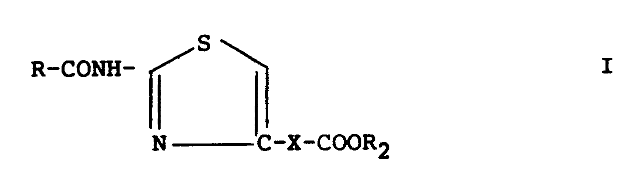 wherein R signifies a group of the formula 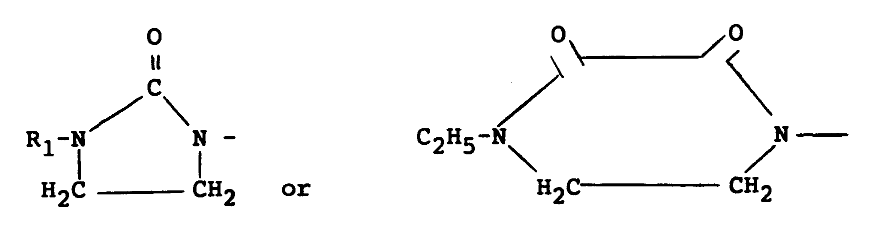 and R₁ represents hydrogen or CH₃SO₂, R₂ represents hydrogen or a low alkyl residue and X represents 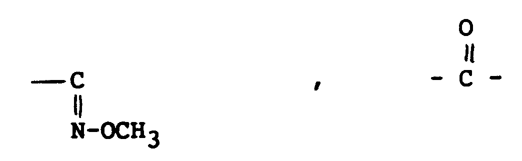 or -CH₂-

2. A process for the production of the compounds according to claim 1, characterised in that a compound of the general formula 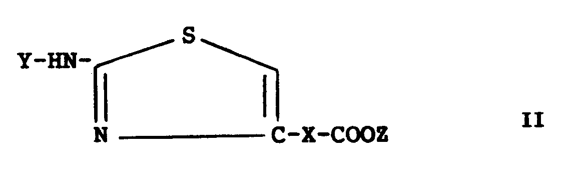 in which X has the signification as stated above, Z represents a low alkyl residue or -Si(CH₃)₃ and Y represents -H or the group -Si(CH₃)₃, is acylated with compounds of the formula
RCOCl III
wherein R has the significations as stated above.

3. A process according to claim 2, characterised in that the acylation is carried out in water-free organic solvents such as halogenated hydrocarbons or esters of low fatty acids.

4. A process according to claim 2, characterised in that the acylation is carried out in the presence of a hydrogen chloride acceptor.

5. A process according to claim 2, characterised in that the acylation is carried out at a temperature of 0° C up to the boiling temperature of the solvent.

## Revendications

1. Dérivés acyluréidoaminothiazolylés, représentés par la formule générale : 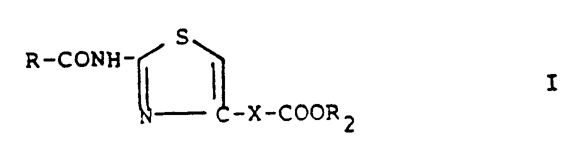 dans laquelle :
- R représente un groupe de la formule : 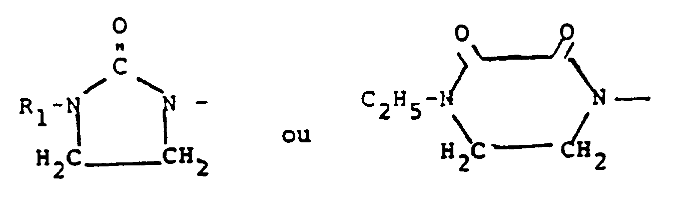 et R₁ représente hydrogène ou CH₃SO₂ ;
- R₂ représente hydrogène ou un reste alkyle inférieur ; et
- X représente le groupe 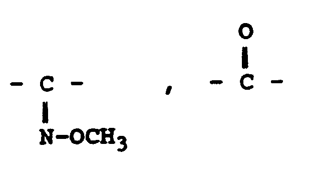 ou -CH₂- .

2. Procédé de fabrication des composés tels que définis à la revendication 1, caractérisé par le fait que l'on conduit l'acylation d'un composé représenté par la formule générale : 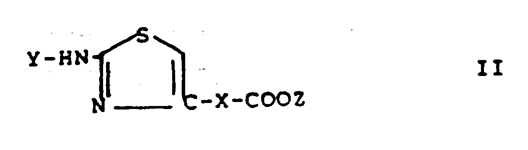 dans laquelle :
- X a les significations ci-dessus ;
- Z représente un reste alkyle inférieur ou -Si(CH₃)₃ ; et
- Y représente -H ou le groupe -Si(CH₃)₃,
par des composés représentés par la formule :
RCOCl (III)
où R possède les significations ci-dessus.

3. Procédé selon la revendication 2, caractérisé par le fait que l'on conduit l'acylation dans des solvants organiques anhydres, comme des hydrocarbures halogénés ou des esters d'acides gras inférieurs.

4. Procédé selon la revendication 2, caractérisé par le fait que l'on conduit l'acylation en présence d'un accepteur de chlorure d'hydrogène.

5. Procédé selon la revendication 2, caractérisé par le fait que l'on conduit l'acylation à une température de 0°C jusqu'à la température d'ébullition du solvant.
